# EUROPÄISCHE PATENTSCHRIFT

(11) **EP 0 539 536 B1**
(45) Veröffentlichungstag und Bekanntmachung des Hinweises auf die Patenterteilung: **28.08.1996**
(21) Anmeldenummer: 92907982.0
(22) Anmeldetag: 07.04.1992
(51) Int. Cl.: A61C 8/00

(54) **SCHRAUBENEINHEIT**
SCREW UNIT
ENSEMBLE VIS

(30) Priorität: 13.05.1991 DE 4115961
(43) Veröffentlichungstag der Anmeldung: 05.05.1993
(73) Patentinhaber: ALTATEC Medizintechnische Elemente GmbH & Co. KG., 75449 Wurmberg (DE); IMZ-Fertigungs- und Vertriebsgesellschaft für dentale Technologie mbH, D-70794 Filderstadt (DE)
(72) Erfinder: DÜRR, Walter, D-7537 Remchingen (DE); KIRSCH, Axel, D-7024 Filderstadt (DE)
(74) Vertreter: Goddar, Heinz J., Dr.
(86) Internationale Anmeldenummer: DE9200290
(87) Internationale Veröffentlichungsnummer: WO9220297

(56) Entgegenhaltungen:
- EP-A- 0 291 103
- WO-A-88/08283
- CH-A- 413 224

## Beschreibung

Die Erfindung betrifft eine Schraubeneinheit nach dem Oberbegriff von Anspruch 1.

Enossale Implantate, d.h. in einen Kieferknochen einzupflanzende Körper, sind z.B. aus der EP 0 216 031 A1 bekannt und haben sich in der Praxis bereits hervorragend bewährt. Beim Implantieren derartiger Körper muß auf absolute Sterilität geachtet werden, um die Gefahr von Entzündungen niedrig zu halten. Da ein implantierter Grundkörper vor dem Einbringen des Implantatpfostens oder dergleichen zunächst in den Knochen einwachsen muß, wird das Innengewinde des Grundkörpers während dieser Zeit verschlossen, da es sie sich sonst mit totem Material füllen würde, das nicht abtransportiert werden kann.

Zum Einbringen des Grundkörpers in den Knochen ist zu dessen besserer Handhabung gewöhnlich ein Einbringpfosten in das Innengewinde eingeschraubt, der anschließend wieder entfernt wird. Danach wird das Innengewinde des Grundkörpers mit einer speziellen Verschlußschraube verschlossen. Diese Schraube wird entfernt, sobald der Grundkörper fest eingewachsen ist und ein Implantatpfosten, eine Implantatextension, ein Pfosten für Attachments und dgl. oder eine Schraube zur Befestigung von Kronen und anderen Aufbauten in der Zahnprothetik, in den implantierten Grundkörper eingeschraubt werden soll.

Aus WO88/08283 ist ein Werkzeug zum Einschrauben eines mit Schraubgewinde versehenen prothetischen Teils bekannt, bei dem das Werkzeug mit dem einzuschraubenden prothetischen Teil in festem, aber lösbarem Sitz verbunden ist.

Es ist Aufgabe der Erfindung, eine Möglichkeit zu schaffen, einen mit Verschlußschraube für das Innengewinde versehenen im Knochen zu implantierenden Grundkörper, während der Implantation sicher und präzise zu handhaben.

Erfindungsgemäß wird diese Aufgabe durch eine Schraubeneinheit mit den Merkmalen des kennzeichens von Anspruch 1 gelöst. Ein solcher Pfosten, an den man mit entsprechendem Spezialwerkzeug, wie z.B. einer Pinzette, angreifen kann, bietet die Möglichkeit einer sicheren und präzisen Handhabung des zu implantierenden Grundkörpers. Dabei kann er nach dem Einbringen des Grundkörpers ohne weiteres von der Verschlußschraube gelöst und entfernt werden. Das Innengewinde bleibt während des gesamten Zeitraums der chirurgischen Manipulation aseptisch verschlossen. Der Einbringpfosten kann dabei zusammen mit der Schraube sterilisiert werden, und verbleibt so lange in seinem Sitz an der Verschlußschraube, wie er für die Manipulation des Grundkörpers benötigt wird.

Die erfindungsgemäße Schraubeneinheit hat den Vorteil, daß man in Rotationsrichtung eine fast beliebig große Kraft aufwenden kann, während schon eine relativ geringe Kraft ausreicht, um in axialer Richtung den Einbringpfosten aus der Verbindung mit der Verschlußschraube zu lösen.

Es wird weiter empfohlen, eine Verschlußschraube zu verwenden, die einen Schraubschlitz aufweist, der vor dem Rand des Schraubenkopfes endet, und den Einbringpfosten mit wenigstens einer Zunge versehen, an der mindestens eine Ausballung vorgesehen ist, die unter Klemmwirkung im Schraubschlitz ruht, wenn der Einbringpfosten und die Verschlußschraube zusammengesteckt sind. Durch einen derartigen Schraubschlitz und eine damit in Eingriff kommende Zunge am Einbringpfosten ist auf besondere und wirkungsvolle Weise der angestrebte reibschlüssige Kontakt zwischen Einbringpfosten und Verschlußschraube zu erreichen. Eine solche Ausbildung ist darüber hinaus vorteilhaft, weil ein derartiger Schraubschlitz auch zum sicheren Ansetzen eines herkömmlichen Schraubendrehers dienen kann, wie er z.B. beim Herausdrehen der Schraube aus dem implantierten Grundkörper nach dessen Einwachsen in den Knochen verwendet wird. Ein Abrutschen des Schraubendrehers und damit die Gefahr einer Verletzung des Umgebenden Gewebes kann so zuverlässig verhindert werden. Es entstehen durch eine derartige Ausgestaltung keine zusätzlichen Ecken oder Kanten, in denen sich eventuell Entzündungen bilden könnten. Außerdem wird eine glatte, runde Außenseite der Schraube gewährleistet, die nicht übermäßig das umgebende Gewebe reizt. Schließlich ist auch ein Einwachsen des Gewebes in den Schraubschlitz nicht möglich.

In einer bevorzugten Ausführung wird die Zunge eine Ausnehmung um die Achse des Einbringpfostens herum aufweisen. Durch eine derartige Ausnehmung kann (können) die Ausballung(en) in federnden und klemmenden Kontakt mit den Enden des Schraubschlitzes treten.

Es wird weiterhin vorgeschlagen, eine solche Ausnehmung V-förmig auszubilden. Eine derartige Gestaltung der Ausnehmung hat sich als vorteilhaft erwiesen, da die beiden verbleibenden Abschnitte der Zunge auf diese Weise ohne große Anforderungen an das für die Zunge verwendete Material in den Schraubschlitz einschnappen können.

Bezüglich der Ausbildung der Ausballung(en) wird vorgeschlagen, daß diese mit Preßkanten versehen ist (sind), so daß sich in einem Schnitt entlang der Längserstreckung der Zunge eine trapezoide Form der Ausballung ergibt, wobei bevorzugt eine erste Kante von der rechtwinklig zur Achse des Einbringpfostens verlaufenden Abschlußkante der Zunge zu einer zweiten, parallel zur Achse des Einbringpfostens verlaufenden seitlichen Kante verläuft und dort mit steilem Winkel anschließt, und eine dritte Kante von der seitlichen zweiten Kante unter einem flachen Winkel nach innen zum Ansatzpunkt der Zunge am Einbringpfosten verläuft. Eine derartige Ausgestaltung schafft durch eine wesentlich kleinere Auflagefläche, nämlich nur der seitlichen, zweiten Kante, an den Enden des Schraubschlitzes, gleichzeitig einen ausreichenden Reibschluß und eine gute Lösbarkeit zwischen Verschlußschraube und Einbringpfosten.

Vorzugsweise ist der Schraubschlitz an seinen Enden abgerundet, so daß der Reibschluß in dieser bevorzugten Ausführungsform der Erfindung an lediglich vier Punkten des Schraubschlitzes auftritt, was bei gleichzeitig guter Klemmwirkung ein Herauslösen des Einbringpfostens nach Abschluß der knochenchirurgischen Manipulationen erleichtert.

Dabei spielt insbesondere die V-förmige Ausnehmung eine große Rolle, da erst sie den Preßkanten bzw. -ecken der Ausballungen auf den stehengeblieben Zungenabschnitten die ausreichende Rückstellkraft verleiht. Zusammen mit der speziellen Formgebung der Preßkanten der Ausballung und ggf. des Schraubschlitzes ergibt sich so die Möglichkeit, den Einbringpfosten nach erfolgreichem Einbringen des Grundkörpers einfach zu lösen, d.h. durch Kippbewegungen herauszuhebeln.

Zur besseren Handhabung des Einbringpfostens wird empfohlen, ihn an seinem von der Verschlußschraube entfernten Ende mit einem halbkugelförmigen Schlagkopf zu versehen. Dieser Kopf kann dadurch auch schräg auf ihn auftreffende Schläge zum weiteren Eintreiben des Grundkörpers in den vorbereiteten Knochen aufnehmen.

Weiter wird vorgeschlagen, daß der Einbringpfosten einen vor dem Schlagkopf angeordneten Griffabschnitt versehen mit kleinerem Radius aufweist. An diesem Griffabschnitt kann entsprechendes Spezialwerkzeug zur Manipulation des zu implantierenden Grundkörpers, wie beispielsweise eine Pinzette, sicher angesetzt werden.

Dabei ist benachbart zur Verschlußschraube am Einbringpfosten bevorzugt noch eine Verbreiterung vorgesehen, um ein Abrutschen des am Griffabschnitt angreifenden Werzeugs auch zum Knochen hin zu verhindern.

Weitere Merkmale und Vorteile der Erfindung ergeben sich aus der nachfolgenden Beschreibung, in der ein Ausführungsbeipiel anhand der beliegende Zeichnung im einzelnen erläutert ist. Dabei zeigt:
- Fig. 1: die Verschlußschraube mit dem an ihr befestigten Einbringpfosten,
- Fig. 2: eine Schnittdarstellung entlang der Linie A-A in Fig. 1,
- Fig. 3: eine Draufsicht auf den Kopf der Verschlußschraube mit Schraubschlitz, und
- Fig. 4: die Zunge des Einbringpfostens in einer Ausschnittsdarstellung.

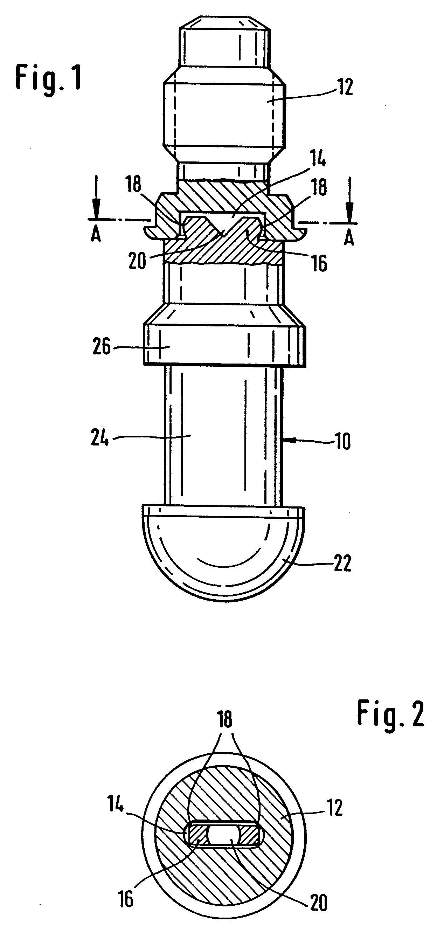

Fig. 1 zeigt eine Verschlußschraube 12 mit eingesetztem Einbringpfosten 10, wie sie für das Implantieren eines mit der Verschlußschraube verschlossenen Grundkörpers miteinander verbunden sind. Der Schraubenkopf der Verschlußschraube 12 ist dabei mit einem Schraubschlitz 14 versehen, der nicht durchgehend ausgebildet ist, sondern vor dem Rand des Schraubenkopfes endet. Wie bereits oben näher erläutert, dient er zur Aufnahme einer Zunge 16 am Einbringpfosten 10, die mit zwei Ausballungen 18 versehen ist, zwischen denen eine V-förmige Ausnehmung 20 angeordnet ist, deren obere Kanten - wie in Fig. 2 zu erkennen - gebogen ausgebildet sind.

Der Schraubschlitz 14 im Kopf der Schraube 12 ist darüberhinaus - wie ebenfalls in Fig. 2 erkennbar - mit abgerundeten Enden versehen, so daß es im wesentlichen lediglich zu einem Reibschluß zwischen den vier Eckpunkten der beiden Ausballungen 18 der Zunge 16 und dem Schraubschlitz 14 kommt. Durch die V-förmige Ausnehmung 20 zwischen den beiden Ausballungen 18 kann die Zunge 16, die vorzugsweise aus einem Material mit entsprechenden Elastizitätseigenschaften besteht, in den Schraubschlitz 14 der Verschlußschraube 12 einschnappen und nach Abschluß der knochenchirurgischen Manipulationen in einfacher Weise wieder aus dem festen Sitz an der Verschlußschraube 12 gelöst werden.

In Fig. 1 ist darüberhinaus auch eine besonders bevorzugte Ausführungsform des Einbringpfostens 10 zu erkennen, bei der dieser an seinem von der Verschlußschraube 12 entfernten Ende mit einem halbkugelförmigen Schlagkopf 22 versehen ist, der als Schlagfläche dient, um dadurch den zu implantierenden Körper weiter in den vorbereiteten Knochen einzutreiben, wobei durch die Halbkugelform auch schräg auftreffende Schläge aufgenommen werden können. In Richtung auf die Verschlußschraube 12 folgt im Anschluß an den Schlagkopf 22 ein Griffabschnitt 24 mit kleinerem Radius. Dieser Griffabschnitt dient zum Angriff eines Spezialwerkzeugs zur Manipulation der erfindungsgemäßen Schraubeneinheit, wie beispielsweise einer Pinzette. Dabei ist durch den größeren Radius des Schlagkopfes 22 sichergestellt, daß das entsprechende Spezialwerkzeug nicht während der Manipulation nach oben abrutschen kann. Um möglichst auch ein Abrutschen nach unten hin, d.h. zum zu implantierenden Körper und dem diesen umgebenden Knochen- und Gewebematerial hin, zu verhindern, ist benachbart zur Zunge 16 noch eine Verbreiterung 26 am Einbringpfosten 10 vorgesehen.

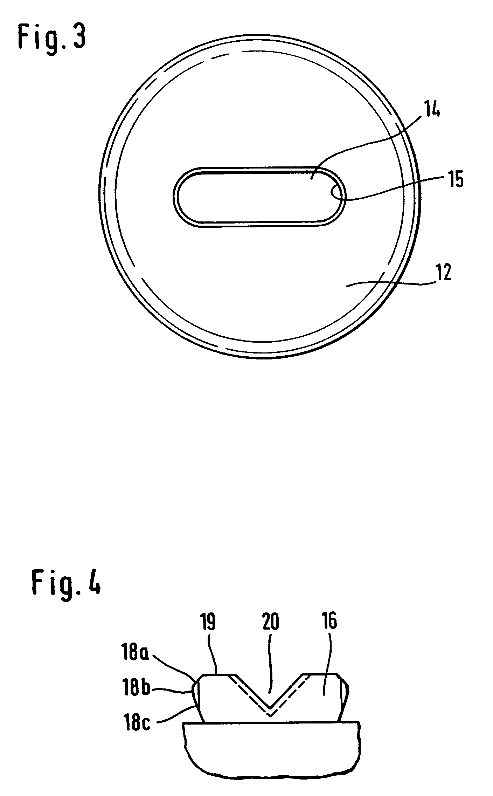

In Fig. 3 ist noch einmal in Draufsicht der Schraubschlitz 14 im Kopf der Schraube 12 dargestellt, wobei hier besonders gut die abgerundeten Enden 15 desselben erkennbar sind.

Die trapezoide Form der Ausballungen 18 durch die Anordnung der Preßkanten 18a, 18b und 18c der Zunge 16 zueinander bezüglich deren Abschlußkante 19 ist in Fig. 5 dargestellt, die einen wesentlich vergrößerter Ausschnitt von Fig. 1 bezüglich der Zunge 16 zeigt. Die Kante 18a dient dabei insbesondere dazu, beim Zusammenstecken der beiden Teile der erfindungsgemäßen Schraubeneinheit, nämlich des Einbringpfostens 10 und der Verschlußschraube 12, eine Führung zu übernehmen. Die relativ kurze Kante 18b erlaubt es, den Einbringpfosten 10 relativ leicht wieder von der Verschlußschraube 12 zu trennen, indem in Richtung des Schraubschlitzes 14 Kippbewegungen des Einbringpfostens 10 relativ zur Schraube 12 vorgenommen werden, die dazu führen, daß die Zunge 16 sich aus dem Schraubschlitz 14 löst.

Durch den so beschriebenen Einbringpfosten 10, der zusammen mit dem mit Verschlußschraube abgeschlossenen Grundkörper sterilisiert werden kann, wird die Möglichkeit geschaffen, den gesamten zu implantierenden Grundkörper zusammen mit der mit der Verschlußschraube 12 in einfacher Weise beim Implantieren auszurichten ohne zusätzliche Manipulationen an der Schraube 12 oder dem zu implantierenden Grundkörper vornehmen zu müssen.

## Patentansprüche

1. Schraubeneinheit mit einer einen Schraubschlitz aufweisenden Verschlußschraube (12) zum aseptischen Verschluß eines Innengewindes in einem in Knochenmaterial zu implantierenden Grundkörper und einem Einbringpfosten (10) in festem, aber lösbarem Sitz an der Verschlußschraube (12), wobei der Einbringpfosten (10) mit der Verschlußschraube (12) in Rotationsrichtung in formschlüssigem und in axialer Richtung in reibschlüssigem Kontakt steht.

2. Schraubeneinheit nach Anspruch 1, dadurch gekennzeichnet, daß die Verschlußschraube (12) einen Schraubschlitz (14) aufweist, der vor dem Rand des Schraubenkopfes endet, und der Einbringpfosten (10) mit wenigstens einer Zunge (16) versehen ist, an der mindestens eine Ausballung (18) vorgesehen ist, die unter Klemmwirkung im Schraubschlitz (14) ruht, wenn der Einbringpfosten (10) und die Verschlußschraube (12) zusammengesteckt sind.

3. Schraubeneinheit nach Anspruch 2, dadurch gekennzeichnet, daß die Zunge (16) eine Ausnehmung (20) um die Achse des Einbringpfostens (10) herum aufweist.

4. Schraubeneinheit nach Anspruch 3, dadurch gekennzeichnet, daß die Ausnehmung (20) im wesentlichen V-förmig ausgebildet ist.

5. Schraubeneinheit nach einem der vorangehenden Ansprüche, dadurch gekennzeichnet, daß die Ausballung(en) (18) an der Zunge (16) mit Preßkanten (18a-c) versehen ist (sind), so daß sich in einem Schnitt entlang der Längserstreckung der Zunge (16) eine trapezoide Form der Ausballung(en) (18) ergibt.

6. Schraubeneinheit nach Anspruch 5, dadurch gekennzeichnet, daß eine erste Kante (18a) von der rechtwinklig zur Achse des Einbringpfosten (10) verlaufenden Abschlußkante (19) der Zunge (16) zu einer zweiten, parallel zur Achse des Einbringpfostens (10) verlaufenden seitlichen Kante (18b) verläuft, und dort mit steilen Winkel anschließt, und eine dritte Kante (18c) von der seitlichen zweiten Kante (18b) unter einem flachen Winkel nach innen zum Ansatzpunkt der Zunge an den Einbringpfosten (10) verläuft.

7. Schraubeneinheit nach einem der Ansprüche 1 bis 5, dadurch gekennzeichnet, daß der Schraubschlitz (14) an seinen Enden (15) abgerundet ist.

8. Schraubeneinheit nach einem der vorangehenden Ansprüche, dadurch gekennzeichnet, daß der Einbringpfosten (10) an seinem von der Verschlußschraube (12) entfernten Ende mit einem halbkugelförmigen Schlagkopf (22) versehen ist.

9. Schraubeneinheit nach einem der vorangehenden Ansprüche, dadurch gekennzeichnet, daß der Einbringpfosten (10) einen vor dem Schlagkopf (22) angeordneten Griffabschnitt (24) mit kleineren Radius aufweist.

10. Schraubeneinheit nach einen der vorangehenden Ansprüche, dadurch gekennzeichnet, daß benachbart zur Verschlußschraube (12) am Einbringpfosten (10) eine Verbreiterung (26) vorgesehen ist.

## Claims

1. Screw unit with a screw plug (12) comprising a screw slot for aseptically occluding an internal thread in a basic body to be implanted in bone material and with an insertion post (10) in a firm yet releasable fit at the screw plug (12), wherein the insertion post (10) is in positive contact with the screw plug (12) in the direction of rotation and in frictional contact with it in the axial direction.

2. Screw unit according to claim 1, characterised in that the screw plug (12) comprises a screw slot (14) which ends before the edge of the screw head, and the insertion post (10) is provided with at least one tongue (16) on which at least one bulge (18) is provided, which bulge rests in the screw slot (14) under a clamping action when the insertion post (10) and the screw plug (12) are fitted together.

3. Screw unit according to claim 2, characterised in that the tongue (16) comprises a recess (20) around the axis of the insertion post (10).

4. Screw unit according to claim 3, characterised in that the recess (20) is essentially V-shaped.

5. Screw unit according to one of the preceding claims, characterised in that the bulge(s) (18) on the tongue (16) is (are) provided with press edges (18a-c), so as to produce a trapezoidal shape of the bulge(s) (18) in a section along the longitudinal extent of the tongue (16).

6. Screw unit according to claim 5, characterised in that a first edge (18a) extends from the terminal edge (19), which extends at a right angle to the axis of the insertion post (10), of the tongue (16) to a second, lateral edge (18b), which extends parallel to the axis of the insertion post (10), and adjoins here at a steep angle, and a third edge (18c) extends inwards from the lateral second edge (18b) to the starting point of the tongue at the insertion post (10) at a shallow angle.

7. Screw unit according to one of claims 1 to 5, characterised in that the screw slot (14) is rounded at its ends (15).

8. Screw unit according to one of the preceding claims, characterised in that the insertion post (10) is provided with a hemispherical impact head (22) at its end which is remote from the screw plug (12).

9. Screw unit according to one of the preceding claims, characterised in that the insertion post (10) is provided with a gripping section (24) having a smaller radius and disposed before the impact head (22).

10. Screw unit according to one of the preceding claims, characterised in that a widened part (26) is provided adjacent to the screw plug (12) on the insertion post (10).

## Revendications

1. Ensemble formant vis comportant une vis de fermeture (12) présentant une fente de vissage et destinée à obturer de manière aseptique un taraudage ménagé dans un corps de base à implanter dans un matériau osseux, et un tenon rapporté (10) solidarisé solidement mais de façon amovible à la vis de fermeture (12), dans lequel le tenon rapporté (10) est en contact avec la vis de fermeture (12) de façon à être solidaire avec celui-ci par conjugaison de formes en rotation et par frottement en direction axiale.

2. Ensemble formant vis selon la revendication 1, caractérisé en ce que la vis de fermeture (12) présente une fente de vissage (14) qui se termine avant le bord de la tête de vis et en ce que le tenon rapporté (10) est muni d'au moins une langue (16) sur laquelle est prévue au moins une protubérance en saillie (18) qui repose avec effet de serrage dans la fente de vissage (14) lorsque le tenon rapporté (10) et la vis de fermeture (12) sont fixés l'un à l'autre.

3. Ensemble formant vis selon la revendication 2, caractérisé en ce que la langue (16) présente un évidement (20) ménagé autour de l'axe du tenon rapporté (10).

4. Ensemble formant vis selon la revendication 3, caractérisé en ce que l'évidement (20) a sensiblement une forme en V.

5. Ensemble formant vis selon l'une des revendications précédentes, caractérisé en ce que la (les) protubérance(s) (18) ménagée(s) sur la langue (16) est (sont) munie(s) de bords de pression (18a-c) de sorte que l'on obtient selon une coupe le long de la direction longitudinale de la langue (16) une forme trapézoïdale des protubérances (18).

6. Ensemble formant vis selon la revendication 5, caractérisé en ce qu'un premier bord (18a) s'étend à partir de l'arête (19) de la langue (16) qui s'étend perpendiculairement à l'axe du tenon rapporté (10), jusqu'à un second bord latéral (18b) s'étendant parallèlement à l'axe du tenon rapporté (10), et se raccorde à cet endroit selon un angle important, et en ce qu'un troisième bord (18c) s'étend vers l'intérieur à partir du second bord latéral (18b) selon un angle petit jusqu'au point de raccordement de la langue avec le tenon rapporté (10).

7. Ensemble formant vis selon l'une des revendications 1 à 5, caractérisé en ce que la fente de vissage (14) est arrondie à ses extrémités (15).

8. Ensemble formant vis selon l'une des revendications précédentes, caractérisé en ce que le tenon rapporté (10) est muni, à son extrémité éloignée de la vis de fermeture (12), d'une tête de percussion (22) en forme de demi-sphère.

9. Ensemble formant vis selon l'une des revendications précédentes, caractérisé en ce que le tenon rapporté (10) présente un tronçon de saisie (24) disposé avant la tête de percussion (22) et présentant un rayon inférieur.

10. Ensemble formant vis selon l'une des revendications précédentes, caractérisé en ce qu'il est prévu un élargissement (26) sur le tenon rapporté (10) au voisinage de la vis de fermeture (12).
